(19) **Europäisches Patentamt / European Patent Office / Office européen des brevets**

(11) **EP 2 519 176 B1**

(12) **EUROPEAN PATENT SPECIFICATION**

(45) Date of publication and mention
of the grant of the patent:
**14.05.2014 Bulletin 2014/20**

(51) Int Cl.:
***A61B 18/20*** (2006.01)      ***A61N 5/06*** (2006.01)

(21) Application number: **10813113.7**

(22) Date of filing: **29.12.2010**

(86) International application number:
**PCT/IB2010/003358**

(87) International publication number:
**WO 2011/080574 (07.07.2011 Gazette 2011/27)**

(54) **DEVICE FOR DERMATOLOGICAL TREATMENT USING A LASER BEAM**

VORRICHTUNG ZUR DERMATOLOGISCHEN BEHANDLUNG MITHILFE EINES LASERSTRAHLS

DISPOSITIF DE TRAITEMENT DERMATOLOGIQUE À L'AIDE D'UN FAISCEAU LASER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.12.2009 FR 0906375**

(43) Date of publication of application:
**07.11.2012 Bulletin 2012/45**

(73) Proprietor: **Vivatech**
**75008 Paris (FR)**

(72) Inventors:
• **GIRAUD, Sylvain**
**F-13120 Gardanne (FR)**

• **GOSSE, Alban**
**F-13105 Mimet (FR)**
• **CORNIL, Alain**
**F-13090 Aix En Provence (FR)**
• **PERONNE, Patrick**
**F-13100 Aix en Provence (FR)**

(74) Representative: **Barbot, Willy**
**SIMODORO**
**Parc Cézanne II, Bât. G**
**290 Avenue Galilée**
**13857 Aix en Provence Cedex 3 (FR)**

(56) References cited:
**US-A- 5 354 323       US-A- 5 662 643**
**US-A1- 2006 041 289       US-B1- 6 190 377**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

### _Field of the invention_

This invention concerns a device for dermatological treatment using a laser beam, preferably a laser beam having a wavelength from 1.1 to 1.6 $\mu$m, such as 1.21 $\mu$m.

### _Prior Art_

It is known via document WO 2009/071592 that a device exists for perioperative and post-operative treatment of surgical wounds. This device uses a laser source with a wavelength from 0.6 $\mu$m to 2.5 $\mu$m, the beam of which is shaped such that, when the laser radiates the skin, the energy distribution over the area to be treated is homogenous. More specifically, the beam is shaped in the form of a rectangle in order to match the geometry of an incision, the incision then being treated in several sections of a size equating the length of the rectangle formed by the laser beam.

The difficulty with this device lies in the fact that the power of the laser and the length of exposure for each section must be determined in order to heat the edges of the incisions to a temperature that must be precisely between 45°C and 55°C (a temperature lower than 45°C being ineffective and a temperature higher than 60°C causing burning). Within this range, heating generates thermal stress in the dermis which is expressed by the production of specific proteins (HSP: heat shock proteins) intervening in the natural healing mechanism. Heating therefore promotes more rapid and better organised regeneration of the tissue that has been subject to incision. Finally, healing is thus facilitated and is, in the end, less visible.

In order to limit the risk of exceeding 60°C and burning the patient, the laser can be combined with a safety strip of the type described in document WO 2008/107563. This strip ensures users' safety, because the laser can only be activated when close to it (for example less than 5 mm). In order to modulate the firing power of the laser, there are in addition different types of strips according to the patient's skin type. In fact, skin types can be grouped into categories (six according to the Fitz-Patrick test: Phototype I for very white skin to Phototype VI for very black skin). The practitioner therefore chooses the strip associated with the patient's phototype, the strip permitting the laser device to adjust its treatment parameters (power, time) to the patient's phototype.

The inventors showed that the use of a laser source having a wavelength of 1210 nm instead of 810 nm enable heating with nearly no influence of the skin type.

However, the patient's phototype is not the only parameter that influences the temperature reached (final temperature) during treatment by the laser device; the following variables also have an influence:

- The temperature of the zone before treatment by the laser (initial temperature).

- The presence of blood in the incision, in the edges of the incision and/or under the incision exerts a strong influence because blood is heated more rapidly than lightly pigmented skin. Of course, this factor depends significantly on the type of operation and the practitioner's surgical technique. The inventors demonstrated that at a wavelength of 1210 nm, pure blood and a bloodless skin surface are subject to more or less the same heating. The quantity of blood present in the incision will exert a weak influence on the heating of the area irradiated by the laser and will therefore have a weak influence on the treatment of the scar.

- The vascularisation of the irradiated tissue. The more the tissue is irrigated by heated blood, the more rapidly it will heat, still due to the fact that blood absorbs heat more quickly compared with other components of the skin.

- The thickness of the skin that has been subject to incision. Presently, the inventors observed that the heating obtained with a laser source having a wavelength of 1210 nm is homogeneous to a depth of at least 2 mm.

These parameters are difficult to measure during routine use of the device and may be extremely variable depending on the patient and the practitioners' techniques. As a consequence, it is difficult to predict accurately the temperature reached during treatment using the laser device. Hence, the risk of exceeding 60° C (burning) or of the temperature remaining below 45° C (ineffective treatment) cannot be excluded.

In order to mitigate similar difficulties, the prior art has envisaged controlling the temperature rise in the skin by placing the laser device under the control of an infrared sensor (pyrometer) that monitors the temperature of the patient's skin in real-time, and, if necessary, modulates' the power of the laser beam on the skin. A schematic diagram of such devices is envisaged in particular in US 5,409,481, US 2007/0179484, and US 2006/0041289 A1.

It is in fact known that contactless measurement of the temperature of any body can be achieved by measuring the infrared radiation emitted by this body. In fact, according to Planck's law, every body emits infrared radiation, the wavelength and power of which being linked to temperature. For example, a body at 300°K (23°C) essentially emits infrared radiation within a wavelength range from 6 $\mu$m to 10 $\mu$m.

For a body having a temperature $T_{obj}$, the power $E$ radiated per unit area is given by:

$$E = \varepsilon * \sigma * T_{obj}^4$$

where ε is the emissivity of the body under consideration and where σ is the Stefan-Boltzmann constant.

The wavelength of the laser source used by dermatological treatment devices is within the range 0.6 μm to 2.5 μm. The temperatures to be measured are lower than 70°, corresponding to heat radiation within the range of 6 μm to 10 μm. Since the two ranges do not overlap, temperature measurement in the presence of laser radiation is therefore possible.

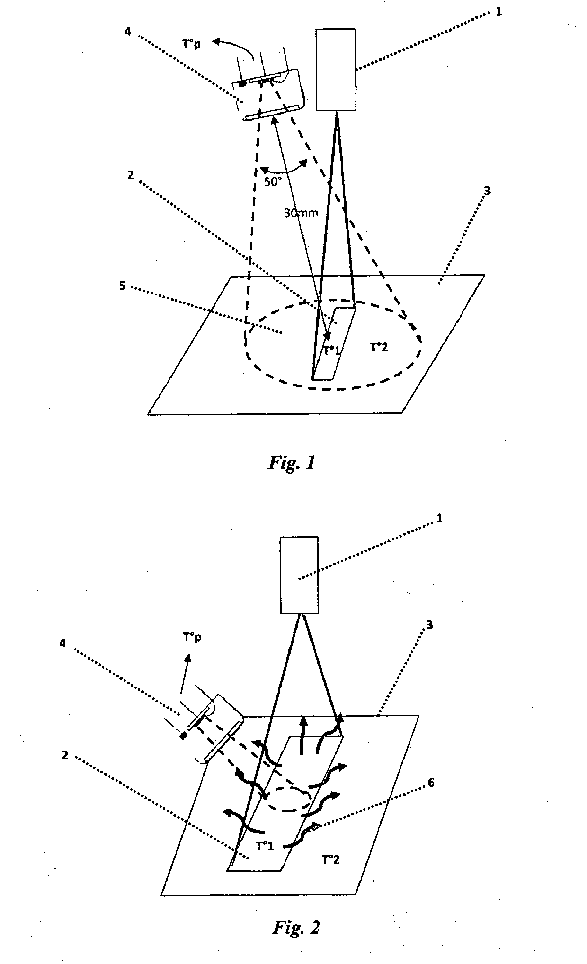

In the first place, the zone heated by the device's laser beam is generally small in size because a great amount of energy per unit area is required to heat the zone to be treated. Figure 1 illustrates this problem.

This figure shows a light source (1) irradiating and therefore heating a zone (2) of the skin (3) to be treated. The shape of the zone (2) may be, for example, circular or rectangular depending on the desired therapeutic application. The area $S_1$ of zone (2) is generally between 0.1 cm² and 2 cm².

This figure also shows the infrared sensor (4) intended to measure the temperature of zone (1). Its field of view on the skin (3) is zone (5). The fields of view of infrared thermal sensors are generally wide, that is a cone of vision, the total typical angle of which is 50° (varying from 20° to 70°, depending on the type). By placing such a sensor at a distance of 30 mm from the zone irradiated by the light beam, the area $S_2$ of zone (5) seen by the infrared sensor equals:

$$S_2 = 3 * \tan\left(\frac{50°}{2}\right) = 6.14 cm^2$$

therefore significantly greater than the area $S_2$ of zone (5).

The result of this is $T°_1 > T°_p > T°_2$, where

> $T°_1$ is the temperature of zone (2) irradiated by the laser beam,
>
> $T°_2$ is the temperature of the skin (3) that is not irradiated by the laser beam,
>
> $T°_p$ is the temperature measured by the infrared sensor.

Figure (11) shows an example of the differences between temperatures $T°_1$ and $T°_p$ in this context.

The temperature measured is therefore extremely imprecise, because it is situated between the temperature of the irradiated zone and that of a non-irradiated zone.

In order to solve this problem, moving the infrared sensor closer to the zone to be treated can be envisaged. However, an additional difficulty then appears, because the skin is a medium that gives rise to strong diffusion of light rays.

The result of this is that, before being absorbed, photons may follow complex routes within the skin. As shown in figure (2), some (6) of these photons are caused to "come out of" skin once more, as if they have been emitted by the latter. In practice, about 10% of the light power incident on the patient is thus diffused and re-emitted outside the skin. This diffusion disturbs the operation of the infrared heat sensor in two different ways:

- The photons (6) resulting from the diffusion of the light beam (wavelength 0.6 μm to 2.5 μm) and the photons radiated by the skin due to its temperature (wavelength 6 μm to 10 μm) merge and add up to each other. The infrared sensors are generally equipped with a wavelength sensitive selective filter in order to suppress this type of disturbance. However, in this case, the power per unit area of the diffusion of the light beam is 10 to 15 times greater than the power per unit area radiated by a body at a temperature of 45° C. The filter, even if it is effective, cannot be sufficient. Temperature measurements are therefore increasingly false as the power of the light beam increases. Compensating for this disturbance via calculation implies finding out the proportion of the power of the light beam that is diffused. However, this value varies from one skin type to another (between white skin and black skin, for example).

- The photons resulting from the diffusion of the light beam have a secondary effect of warming all the elements that are close to the zone undergoing treatment, in particular the temperature sensor. Thus, the temperature measurement determined by the sensor is calculated based on two items of information: the electrical signal returned by the sensor, and measurement of the temperature of the sensor itself. A temperature probe is therefore placed as close as possible to the infrared thermal sensor. Rapid heating such as that created by the diffusion of the light beam creates a transient heat balance between the infrared sensor and the temperature probe. The data from the temperature probe is thus falsified, as is the temperature calculated.

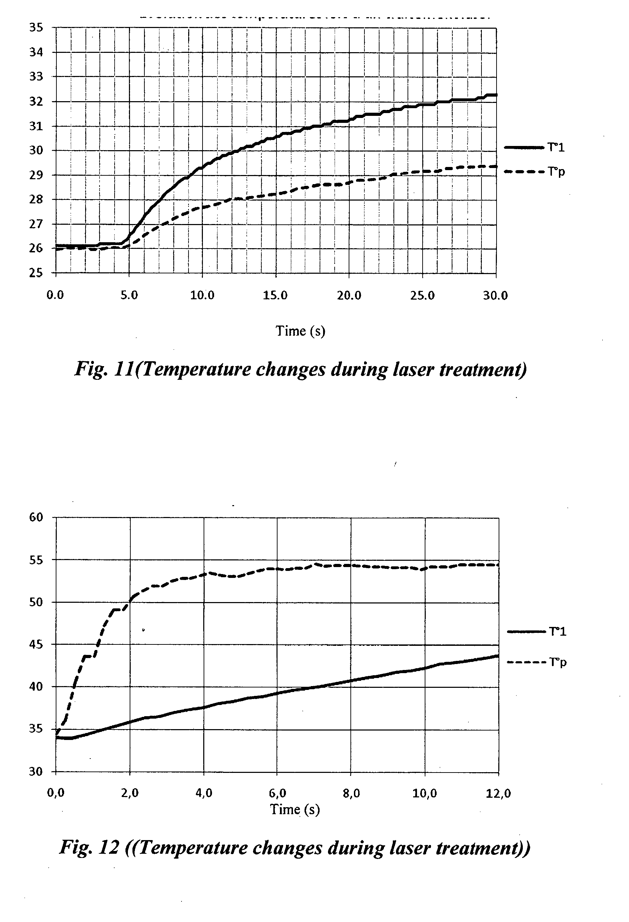

Figure 12 shows an example of the differences between temperatures T°1 and T°p in this context.

In the light of these additional difficulties linked to the distance away from, or proximity to the zone to be treated, none of the laser devices employed in the prior art has in practice been combined with an infrared sensor, in the light of the significant inaccuracy of the measurements.

## Summary of the invention

The invention is defined in the appended set of claims.

The invention aims to mitigate aforementioned drawbacks, and its purpose is to supply a dermatological treatment device using a laser beam that ensures the effectiveness of the treatment, while eliminating risks of burning by using an infrared sensor.

To this end, the first purpose of the invention is to provide a dermatological treatment device using a light beam, comprising:

- a laser light source suitable for directing a laser beam onto at least a zone of the surface of the skin to be treated,

- a means of contactless measurement sensitive to radiation according to temperature, configured to measure the temperature of a skin surface corresponding to the skin zone treated, and

- a means of controlling the aforesaid light source via the aforesaid means of measurement.

The aforesaid means of contactless measurement comprises an infrared sensor and an objective lens suitable for focusing the field of view of the aforesaid infrared sensor, such that the skin surface contained within the aforesaid field of view is wholly included in the area of skin treated by the device.

The purpose of the invention is also to provide a system for dermatological treatment using a laser beam, the system comprising a mechanism as described above and means of interaction between the laser source and the skin zone to be treated, the aforesaid means of interaction being equipped to cooperate with the aforesaid means of control.

## Description of the figures

- Figure 1 illustrates one of the difficulties that needs to be resolved in the context of this invention.

- Figure 2 illustrates another difficulty that needs to be resolved in the context of this invention.

- Figures 3 and 4 illustrate a third difficulty that needs to be resolved in the context of this invention.

- Figure 5 is a diagrammatic representation of a device according to the invention.

- Figure 6 is a diagrammatic representation of an infrared sensor used in the context of this invention.

- Figure 7 is a diagrammatic representation of an initial method of treatment.

- Figure 8 is a diagrammatic representation of a second method of treatment.

- Figure 9 is a diagrammatic representation of a third method of treatment.

- Figure 10 is a diagrammatic representation of a variation of the method shown in figure 9.

- Figure 11 is a representation of the changes over a period of time of two temperatures mentioned in the context of figure 1.

- Figure 12 is a representation of the temporal changes of two temperatures mentioned in the context of figure 2.

- Figure 13 is a representation of the temporal changes of two temperatures mentioned in the context of figures 3 and 4.

- Figure 14 is a representation of the temporal changes of two temperatures mentioned in the context of the embodiment in which the means of control is of the on/off type.

- Figure 15 is a representation of the temporal changes of two temperatures mentioned in the context of the embodiment in which the means of control are of the regulation type.

- Figure 16 is a diagrammatic representation of a dermatological treatment system comprising a device according to the invention; and

- Figure 17 is a diagrammatic representation of the field of view of an infrared sensor of a skin zone treated in the presence of an objective lens.

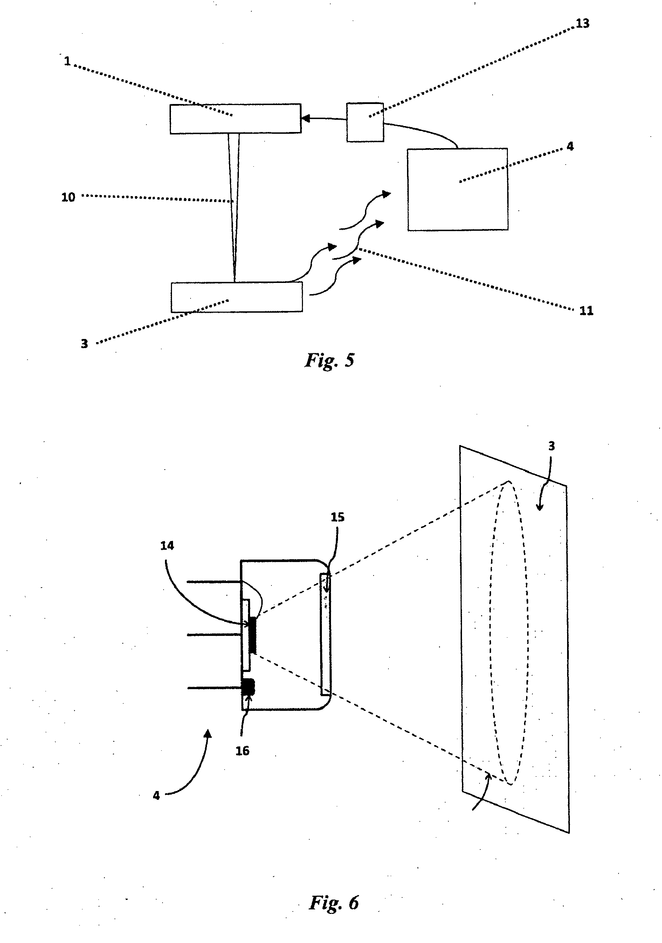

It should be noted that in figures 5 to 17 the same reference figures have been used as those used in figures 1 to 4 for equivalent elements.

## Detailed description of the invention

The laser source emits within the wavelength range from 0.6 $\mu$m to 2.5 $\mu$m, preferably from 0.7 to 2.2 $\mu$m and particularly preferably from 1.1 to 1.6 $\mu$m, such as 1.21 $\mu$m. This laser source permits the delivery, on the skin surface being treated, of a fluence between 1 and 500 J/cm$^2$, preferably a fluence between 2 and 250 J/cm$^2$, and particularly preferably a fluence between 5 and 200 J/cm$^2$.

In the context of the device according to the invention, the field of view of the infrared sensor is not therefore more divergent but has a concentration zone (focal point) around which the diameter of the field of view is minimal. One can thus in practice obtain fields of view with a diameter between 1.5 mm and 8 mm at a distance from the sensor of between 10 mm and 60 mm. It is thus possible to space the sensor from the treatment area, thus avoiding disturbance due to diffusion of the laser light. In fact, the intensity of light disturbance diminishes proportionally to the square of the distance between the sensor and the treatment zone.

This arrangement is also favourable with regard to the first difficulty mentioned above, a field of view of the sensor being greater than the size of the treatment zone, which also considerably falsifies the measurements of the infrared sensor.

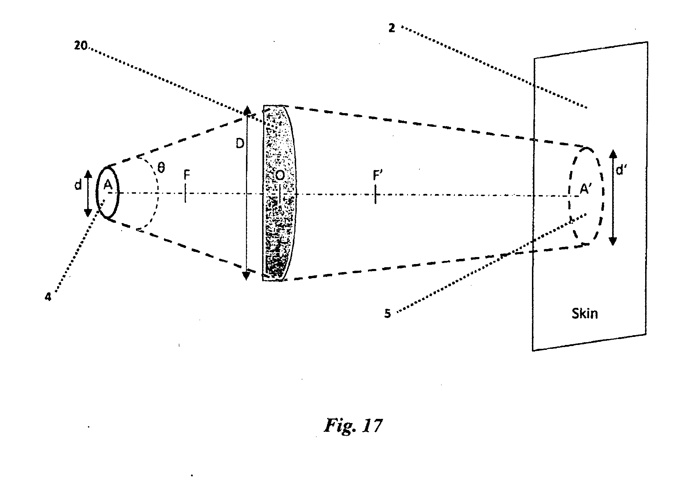

Concerning more specifically the objective lens (20), figure 17 represents the different parameters that need to be incorporated when choosing and positioning this lens in relation to the infrared sensor (4) and the area of skin to be treated (2), such that the skin area contained in the field of view (5) of the infrared sensor is fully included in the zone of skin (2) treated by the dermatological treatment device.

More specifically, in this figure:

* A and d represent the centre and the diameter of the photosensitive element of the infrared sensor (4), respectively. $\theta$ represents the angle of the field of view of the photosensitive element in the absence of the objective lens (2).

* A' and d' represent the centre and the diameter of the image (5) of the photosensitive element on the patient's skin, respectively, the image being fully included in the skin area (2) being treated by the light beam.

* F is the primary focal point of the lens, F' is the secondary focal point of the lens, O is the centre of the lens, and D is the diameter of the objective lens.

More specifically, the inventors have been able to demostrate that the position and the diameter of the objective lens (20) can be determined by solving the following three equations:

1)

$$\frac{1}{\overline{OA'}} - \frac{1}{\overline{OA}} = \frac{1}{f'}$$

2)

$$\frac{\overline{d'}}{\overline{d}} = \frac{\overline{OA'}}{\overline{OA}},$$

and

3)

$$D' = OA * Tan(\theta) + d,$$

where

* OA is the distance between the centre of the photosensitive element of the infrared sensor and the centre of the objective lens, with OA being between 0 and 100 mm, preferably between 5 and 50 mm, and particularly preferably between 10 and 30 mm.

* OA' is the distance between the centre of the objective lens and the surface of the skin to be treated with:

    i) the sum of OA' and OA being greater than or equal to 10 mm, preferably greater than or equal to 30 mm, and particularly preferably greater than or equal to 50 mm, and

    ii) the sum of OA' and OA being less than or equal to 500 mm, preferably less than or equal to 100 mm, and particularly preferably less than or equal to 75 mm.

* d is the diameter of the photosensitive element of the infrared sensor, with d being between 0.1 and 10 mm, preferably between 0.5 and 5 mm, and particularly preferably between 1 and 3 mm.

* $\theta$ represents the angle of the field of view of the photosensitive element of the infrared sensor in the absence of the objective lens, with $\theta$ being between 10 and 80 °, preferably between 15 and 60°, and particularly preferably between 20 and 40°.

* d' is the diameter of the image of the photosensitive element on the patient's skin, for the image to be fully included in the skin surface to be treated by the dermatological treatment device, with:

    i) d' being less than 20 mm, preferably less than 10 mm, and particularly preferably less than 5 mm, and

    ii) d' being greater than 0.5 mm, preferably greater than 1 mm.

* f' is the focal length of the objective lens.

* D is the diameter of the objective lens, with D being between 3 and 100 mm, preferably between 4 and 50 mm, and particularly preferably between 5 and 15 mm.

The diameter and the position determined for the objective lens provide that the field of view of the aforesaid infrared sensor can be focused such that it is fully included in the skin area to be treated.

The inventors have thus been able to demonstrate that a device incorporating an infrared sensor and an objective lens (20), having the specific features described previously, enables an efficient treatment of a patient (temperature greater than 45° C), wherein burns can be avoided (temperature lower than 60° C).

Advantageously, the device includes means of filtration composed of a material transparent in the wavelength range 6 $\mu$m to 10 $\mu$m in order to permit temperature measurement, the aforesaid material being opaque in the wavelength range from 0.6 $\mu$m to 2.5 $\mu$m. As an example of such materials, one could use silicon or germanium, preferably silicon.

In this scenario, the disturbance of the diffusion of the light beam then becomes almost negligible due to the "selective wavelength filter".

In some cases, the aforesaid means of filtration could correspond to the objective lens, in particular when the latter is made from silicon or germanium, preferably silicon.

In one particular embodiment, the means of control is of the on/off type suitable for switching off the light source when the temperature measured by the infrared sensor in the skin zone being treated exceeds a predetermined value.

This embodiment essentially aims at avoiding burns in the zone being treated by interrupting the operation of the light source when the temperature measured reaches a critical threshold. Treatment may eventually be resumed when the temperature drops below a second threshold.

In another particular embodiment, the means of control are of the regulation type, suitable for adjusting the power of the light source in order to maintain the temperature measured by the infrared sensor in the skin zone being treated between two predetermined values.

This embodiment permits regulation of the power of the light source according to the temperature measured in order to maintain this latter temperature at an optimum value, given the particular nature of the treatment to be applied and the characteristics of the skin being treated. It may be combined with the previous embodiment as a safety measure in order to interrupt the operation of the light source if the temperature measured reaches a critical threshold.

An additional difficulty results from the fact that the device is intended to be used on the skin of patients via a head (7) applied directly to the skin, as shown in figure (3). Skin is a flexible medium which deforms easily and the surface of which is rarely flat. In addition, skin flexibility is variable from one zone of the body to another and from one individual to another (e.g. depending on age or corpulence).

Skin deformation also depends on the user, depending on the force with which the head of the device is applied to the patient.

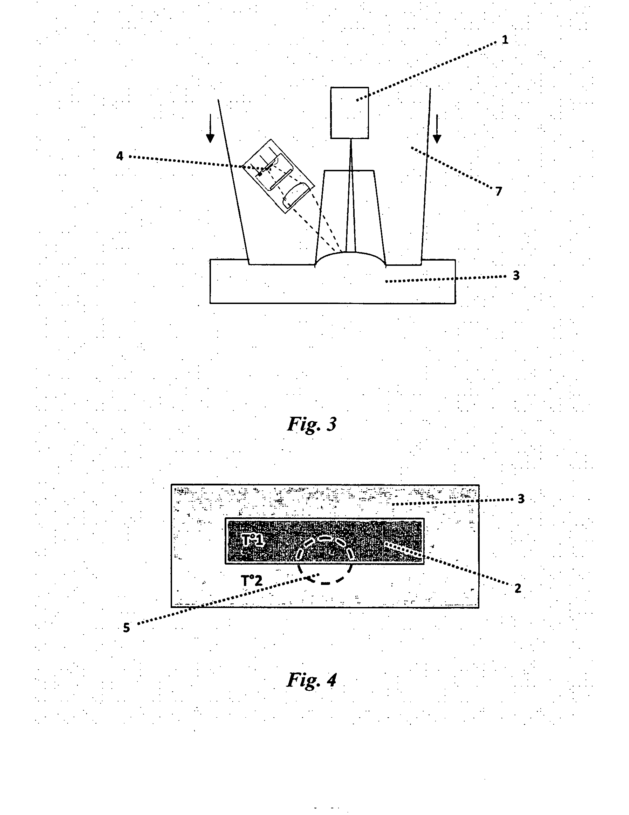

This deformation has three results illustrated in figure 4:

- The skin moves closer to the infrared sensor, reducing the distance between the sensor and the treatment zone and therefore increasing the disturbance of the diffusion of the light beam.

- The device's light beam is divergent, such that the dimensions of the beam increase with the distance it travels. When the skin deforms, the path travelled by the light beam is shorter; the area being treated is smaller and the light energy per surface unit is greater. The dose delivered per surface area will not therefore conform to the dose scheduled by the device.

- The skin is no longer positioned at the point of convergence of the light beam and the sensor's field of view (figure 4). The sensor's field of view (5) can no longer be fully contained within the zone (2) irradiated by the light beam. The temperature measurement will therefore be false.

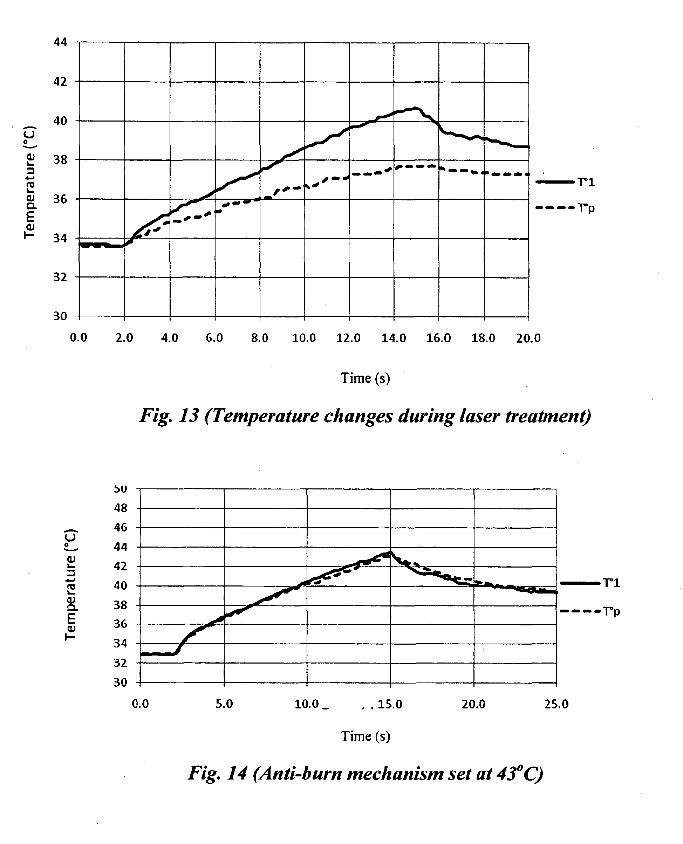

Figure 13 shows the effect of the misalignment between the zone (2) being treated by the light beam and the infrared sensor (12) as shown in figure 4. The temperature measured is not that of the zone irradiated by the light beam:

$$T^\circ_1 > T^\circ_P > T^\circ_2$$

The invention also aims at mitigating this drawback and, to this end, has a mechanism as described previously comprising, in one particular embodiment, a head that can be applied to a part of the skin comprising the area to be treated, in which the aforesaid head comprises a means of smoothing the surface to be treated.

More particularly, the aforesaid head may comprise a cavity equipped with an opening that can be applied to the surface of the skin to be treated, the light beam and the field of view of the means of measurement passing through the aforesaid cavity and arriving in the aforesaid opening, the aforesaid cavity being partially closed by an internal lip peripheral to the aforesaid opening, noticeably flat and which can be applied to the surface of the skin to be treated.

As a variation, the aforesaid opening can be closed with a window made from material that is transparent to the light beam and the radiation detected by the means of measurement.

The purpose of the invention is also to provide a system for dermatological treatment using a laser beam, the aforesaid system comprising a mechanism as described previously and a means of interaction between the aforesaid light source and the skin surface to be treated, the aforesaid means of interaction being equipped to cooperate with the aforesaid means of control.

More particularly, the aforesaid means of interaction may comprise an adhesive medium equipped with means of identification and which can be fixed close to the skin zone to be treated.

The invention permits implementation of a method of dermatological treatment , comprising the following steps:

- Directing the light beam of a device as described previously onto the zone of the skin surface to be treated.

- Measuring, using the means of measurement described previously, the temperature of the skin surface contained within the field of view of the infrared sensor, said skin surface being fully contained within the skin zone to be treated, and

- To control the aforesaid laser source via the aforesaid means of measurement, such that the temperature of the skin zone to be treated is maintained between 45 and 60°C.

We will now describe, by way of a non-exhaustive example, an embodiment referring to the diagrammatic drawings in the appendix.

In figure 5, a device according to the invention comprises a laser source (1) emitting a laser beam (10) in a wavelength range between 0.8 μm and 1.8 μm directed at a part of a patient's skin (3) subject to dermatological treatment.

Depending on its temperature, skin emits known infrared radiation (11) in a wavelength between 6 μm and 10 μm. This infrared heat radiation is detected by a sensor (4) of any known type. The output from the sensor is applied to the input of a regulator (13) which controls the source (1) in terms of power and/or exposure time.

Figure 6 provides a more detailed view of the sensor (4).

The sensor (4) comprises a detector (14) sensitive to infrared heat (thermopile, pyro-electric). A selective wavelength filter (15) (transparent from 3 μm to 12 μm for example) is here added before the detector (14) in order to avoid disturbance by other wavelengths.

The signal returned by the detector (14) is a voltage V in the forum:

$$V = \alpha * \varepsilon * \sigma * (T_{obj}^4 - T_{interne}^4)$$

where $T_{obj}$ is the temperature of the part (5) of the skin (3) placed in the field of view of the sensor, $T_{internal}$ is the internal temperature of the infrared sensor and a is a proportionality constant.

In order to deduce the skin temperature based on the signal from the infrared detector (14), it is necessary to know the sensor's internal temperature. In order to achieve this, a temperature probe (16) is arranged as close as possible to the infrared detector (14), and the signal from the sensor is adjusted in compensation. If

$$T_{interne} = T_{interne\,mesurée}$$

then it is possible to deduce $T_{obj}$ :

$$T_{obj} = \sqrt[4]{\frac{V}{\alpha * \varepsilon * \sigma} + T_{interne\,mesurée}^4}$$

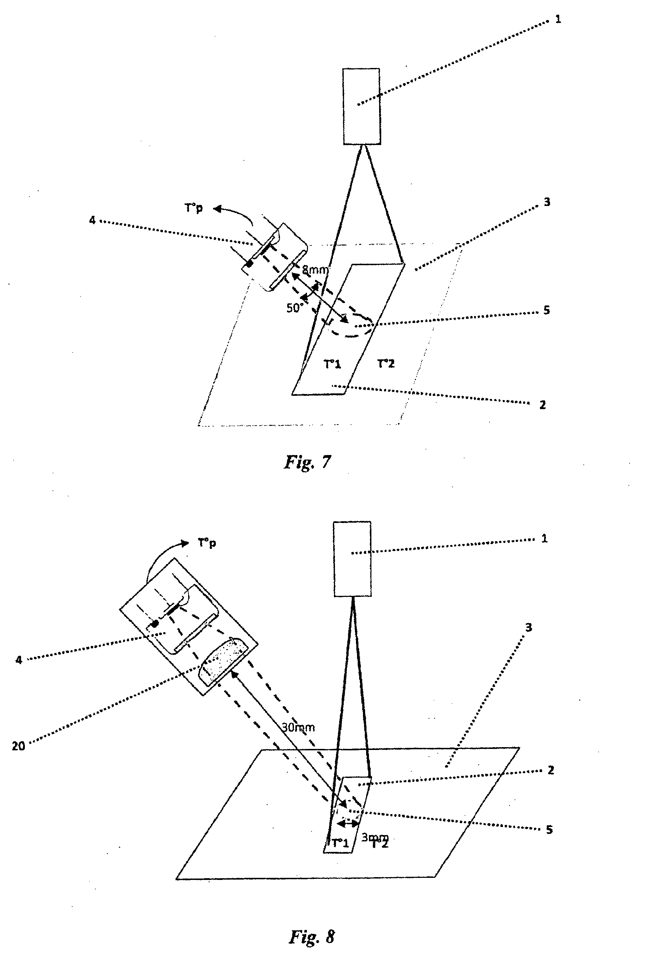

We will now refer to figures 1 and 7.

We have previously described the problems posed by an arrangement of the type shown in figure 1.

In the case of figure 7, a sensor (4) is used, the divergence of the field of view of which is limited. By placing such a sensor as close as possible to the zone being treated (5) (between 5 mm and 10 mm), without obstructing the passage of the light beam, the whole field of view (5) of the sensor is contained in the zone irradiated by the light beam.

The area $S_2$ viewed by the infrared sensor, when it is placed 8 mm from the irradiated zone, is equal to:

$$S_2 = 0.8 * \tan\left(\frac{50°}{2}\right) = 0.44 cm^2$$

In the case of figure 7 where $S_2$ is equal to 0.44cm² it is possible to measure the temperature of zones with an area of more than 0.5 cm² :

$$T°_P = T°_1$$

We will now refer to figures 2 and 8.

In the embodiment shown in figure 8, the sensor (4) has been moved to a position 30 mm from the treatment zone (2) in order to avoid disturbance resulting from the diffusion of the light beam. In order to resolve the problem of the sensor's field of view (5) which is greater than the treatment zone, a convergent lens (20) is used to focus the sensor's field of view.

For example, an infrared sensor (4) combined with an objective lens (20) gives a field of view of 3 mm in diameter at a distance of 30 mm (that is, a measurement area of 0.07 cm$^2$), which permits the temperature measurement of zones with an area of over 0.1 cm$^2$. In the case of figure 8,

$$T^\circ{}_P = T^\circ{}_1$$

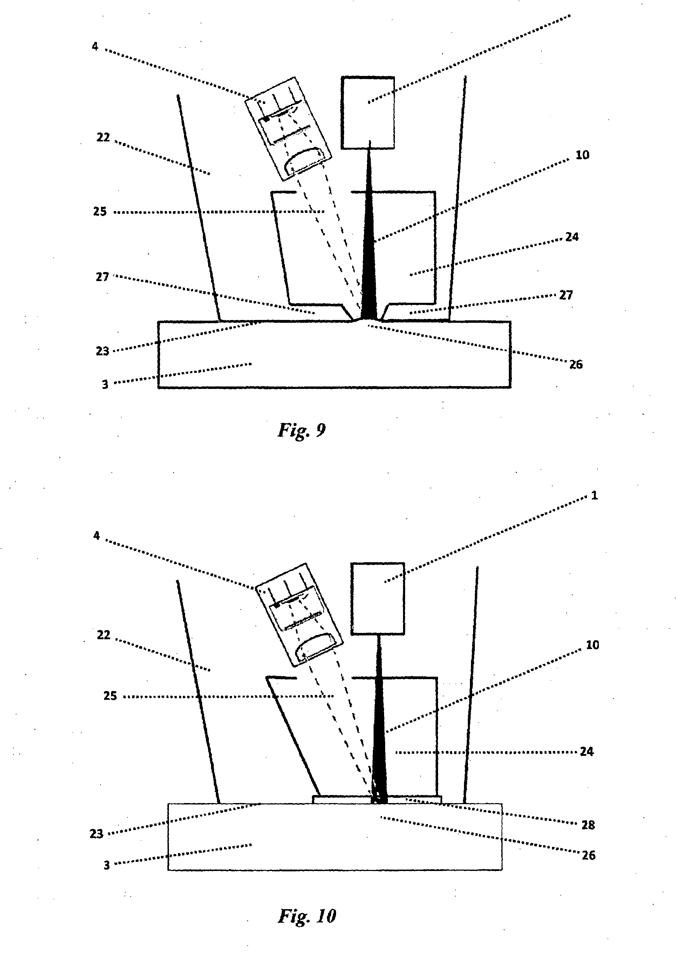

We will now refer to figures 3, 4, 9 and 10. The purpose of the embodiments shown in figures 9 and 10 is to avoid skin creasing likely to affect the temperature measurement.

Here the treatment is applied using a head (22) comprising the laser source (1) and the infrared sensor (4). The head (22) includes a base (23) that comes into contact with the skin (3).

The head (22) in addition forms a cavity (24) traversed by the beam (10) emitted from the laser source (1) and by the field of view (25) of the infrared sensor (4). The cavity (24) has an opening (26) in the base (23), the beam (10) and the field of view (25) arriving in this opening.

In the embodiment shown in figure 9, the base (23) forms a lip (27) surrounding and limiting the opening (26), and therefore partially closing the cavity (24). The opening (26) is of dimensions slightly exceeding those of the light beam.

The surface of the lip (27) external to the cavity (24), as a consequence, limits the formation of skin creases. The more the opening is reduced, the more the skin is kept flat.

In the embodiment shown in figure 10, the opening (26) is blocked by a window (28) made from material that is transparent to the laser beam and the radiation detected by the sensor (4), that is, it has good optical transmission for the wavelengths of the light beam (0.6 μm to 2.5 μm) as well as in the thermal infrared range (6 μm to 10 μm), for example, in calcium fluoride. This window prevents the formation of skin creases. However, this latter embodiment is not really suitable for use with the device when treating wounds, because blood may dirty the window and directly absorb the laser beam.

The invention permits an accurate measurement, in real time and without contact, of the temperature of a zone subject to homogeneous laser radiation (wavelength range: 0.6 μm to 2.5 μm).

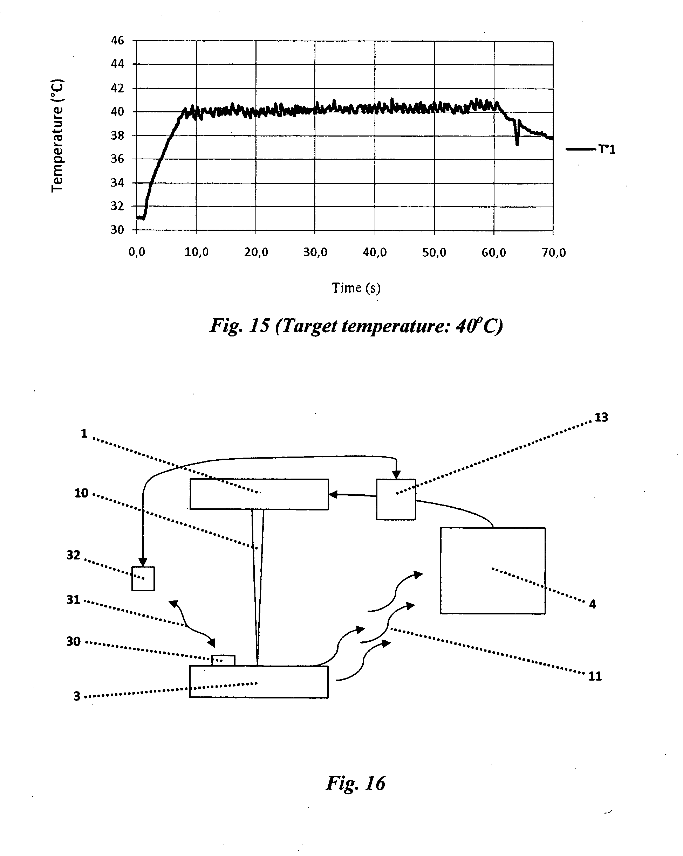

Figures 14 and 15 show the results obtained when:

- The infrared sensor measurement area (4) is contained in the zone (2) irradiated by the light beam (area irradiated is from 0.1 cm$^2$ to 2 cm$^2$).

- The distance between the infrared sensor and the zone irradiated by the laser beam is sufficiently large (from 10 mm to 60 mm) for the sensor (4) not to suffer from the effects of diffusion of the laser beam by the skin.

- The skin is now flat (anti-creasing) and is at the point of convergence of the laser beam and the field of view (4), of the sensor.

Figure 14 illustrates an initial mode of operation in which the means used to measure temperature is used as a safety device to prevent burning: when the temperature exceeds a certain predetermined threshold (for example, between 40° C and 70° C) the light treatment is interrupted (43° C in the figure).

Figure 15 illustrates a second mode of operation in which the means used to measure temperature is used to control the temperature of the irradiated zone dynamically: the device adjusts its power and exposure time parameters to reach a predetermined temperature range (between 40° C and 70° C). Once the predetermined range has been reached, the device is also able to maintain the temperature over time. The temperature chosen is 40° C; once reached, this temperature is maintained for 53 seconds.

Figure 16 represents a dermatological treatment system comprising a device of the type described above.

This system comprises, apart from the elements of the device described above, a means of interaction between the laser source (1) and the surface of the skin to be treated (3), arranged to cooperate with the control mechanism.

This means of interaction here comprises an adhesive medium (30) equipped with a means of identification, communicating via radio frequencies (31) with an interface (32) connected to the control mechanism (13).

Such a means of interaction is disclosed in document WO 2008/107563 and will therefore not be described here in more detail.

**Claims**

1. A device for dermatological treatment using a laser beam, comprising:

    - a laser source (1) suitable for directing a laser beam onto at least a zone (2) of the surface of the skin to be treated, said laser source permitting the delivery on the skin surface being treated of a fluence between 1 and 500 J/cm$^2$ of skin,
    - a means of contactless measurement sensitive to radiation according to temperature, configured to measure the temperature of a skin surface corresponding to the skin zone (2) treated, and

- a means of controlling (13) the aforesaid laser source via the aforesaid means of measurement;
- wherein the aforesaid means of contactless measurement comprises an infrared sensor (4) and an objective lens (20) suitable for focusing the field of view (5) of the aforesaid infrared sensor such that the skin surface contained within the aforesaid field of view (5) is wholly included in the area of skin (2) treated by the laser beam;
- the aforesaid head comprises a cavity (24) equipped with an opening (26) that is configured to be applied to the surface of the skin to be treated, the light beam and the field of view of the means of contactless measurement passing through the aforesaid cavity and arriving in the aforesaid opening, the aforesaid cavity being partially closed by an internal lip (27) peripheral to the aforesaid opening, said lip (27) being substantially flat and configured to be applied to the surface of the skin to be treated,
- wherein said device comprises a means of filtration arranged between the infrared sensor and the surface of the skin being treated and composed of a material transparent in the wavelength range from 6 $\mu$m to 10 $\mu$m in order to permit temperature measurement, and opaque in the wavelength range from 0.6 $\mu$m to 2.5 $\mu$m.

2. The device according to claim 1, wherein the position and the diameter of the objective lens (20) are determined by solving the following three equations:

1)

$$\frac{1}{\overline{OA\prime}} - \frac{1}{\overline{OA}} = \frac{1}{f\prime\prime}$$

2)

$$\frac{\overline{d\prime}}{\overline{d}} = \frac{\overline{OA\prime}}{\overline{OA}},$$

and
3)

$$D = OA * Tan(\theta) + d,$$

where

* OA is the distance between the centre of the photosensitive element of the infrared sensor (4) and the centre of the objective lens (20) with OA being between 0 and 100 mm, preferably between 5 and 50 mm, and particularly preferably between 10 and 30 mm
* OA' is the distance between the centre of the objective lens (20) and the surface of the skin treated (2), with:

    i) the sum of OA' and OA being greater than or equal to 10 mm, preferably greater than or equal to 30 mm, and particularly preferably greater than or equal to 50 mm, and
    ii) the sum of OA' and OA being less than or equal to 500 mm, preferably less than or equal to 100 mm, and particularly preferably less than or equal to 75 mm

* d is the diameter of the photosensitive element of the infrared sensor (4), with d being between 0.1 and 10 mm, preferably between 0.5 and 5 mm, and particularly preferably between 1 and 3 mm
* $\theta$ represents the angle of the field of view (5) of the photosensitive element of the infrared sensor (4) in the absence of the objective lens (20), with $\theta$ being between 10 and 80°, preferably between 15 and 60°, and particularly preferably between 20 and 40°
* d' is the diameter of the image of the photosensitive element on the patient's skin, the image needing to be fully included in the skin surface to be treated (2) by the dermatological treatment device, with:

    i) d' being less than 20 mm, preferably less than 10 mm, and particularly preferably less than 5 mm, and
    ii) d' being greater than 0.5 mm, preferably greater than 1 mm

* f' is the focal length of the objective lens (20).
* D is the diameter of the objective lens (20) with D being between 3 and 100 mm, preferably between 4 and 50 mm, and particularly preferably between 5 and 15 mm;

wherein the diameter and the position determined for the objective lens (20) provide that the field of view (5) of the aforesaid infrared sensor (4) is focusable such that it is fully included in the skin zone (2) being treated by the laser beam.

3. The device according to claim 1 or 2, wherein the wavelength of the laser source is from 1.1 to 1.6 $\mu$m such as 1.21 $\mu$m, to enable a homogenous heating at a depth of at least 2 mm.

4. The device according to either of claims 1 to 3, **characterised by** the fact that the aforesaid means of control (13) is of the on/off type suitable for switching off the laser source (1) when the temperature measured by the infrared sensor (4) on the zone (2) of skin being treated exceeds a predetermined value.

5. The device according to either of claims 1 to 4, **characterised by** the fact that the aforesaid means of control (13) is of the regulation type suitable for adjusting the power of the laser source (1) in order to maintain the temperature measured by the infrared sensor (4) on the zone (2) of skin being treated between two predetermined values.

6. The device according to either of claims 1 to 5, **characterised by** the fact that the aforesaid means of filtration is made from silicon or germanium.

7. A system of dermatological treatment using a laser beam, **characterised by** the fact that it comprises the device according to any of the above claims and a means of interaction (30, 32) between the aforesaid laser source and the area of skin being treated, the aforesaid means of interaction comprising an adhesive medium (30) equipped with a means of identification configured to communicate via radio frequencies (31) with an interface (32) connected with the aforesaid means of control (13).

**Patentansprüche**

1. Ein Gerät für eine dermatologische Behandlung unter verwendung eines Laserstrahls bestehnd aus:

    - Einer Laserquelle (1), die geeignet ist einen Laserstrahl auf mindestens einen Bereich (2) der Hautoberfläche, der behandelt werden muss, gerichtet werden kann. Besagte Laserquelle erlaubt die Übermittlung einer Fluenz auf die hautoberfläche in einem Umfang von 1 bis 500 J/cm$^2$,
    - eine kontaktlosen Messung, die empfindlich auf Strahlung entsprechend der Temperatur reagiert. Sie ist konfiguriert die Temperatur der Hautoberfläche, die der behandelten Hautoberfläche (2) entspricht, zu messen, und
    - eine Kontrolle (13) der erwähnten Laserquelle über die erwähnte Messungsmethode; wobei
    - die zuvor genannte kontaktlose Messung besteht aus einem Infrarotsensor (4) und einer Objektivlinse (20), die so auf das Sichtfeld (5) des zuvor erwähnten infrarotsensors gerichtet werden kann, dass die Hautoberfläche im zuvor erwähnten Sichtfeld (5) vollständig im Bereich der Hautoberfläche (2) liegt, die vom zuvor Laserstrahl behandelt wurden;

    - Der zuvor erwähnte Kopf besteht aus einer Höhlung (24), die mit einer Öffnung (26) versehen ist, die so konfiguriert wurde, dass sie auf die zu behandelnde Hautoberfläche gesetzt werden kann. Der Laserstrahl und das Sichtfeld der kontaktlosen Messung gehen durch die zuvor erwähnte Höhlung und gelangen an die zuvor besagte Öffnung. Die zuvor erwähnte Höhlung wird teilweise von einer internen Lippe (27) verschlossen, die sich peripherisch der zuvor erwähnten Öffnung befindet. Besagte Lippe ist sehr flach und konfiguriert um auf die zu behandelnde Hautoberfläche angewendet zu werden,
    - Wobei besagtes Gerät umfasst einen Filter zwischen dem Infrarotsensor und der behandelten Hautoberfläche, der aus einem transparenten Material in der Wellenlängenbendbrelte zwischen 6μ und 10μ besteht um die Temperaturmessung zu ermöglichen und einem opaken Material mit einer Wellenlängenbandbreite zwischen 0,6 μ und 2,5μ.

2. Das Gerät entsprechend Antrag 1, wobei die Position und der Durchmesser der Objektivlinse (20) durch die Auflösung der folgenden drei Gleichungen bestimmt wird:

    1)

$$\frac{1}{OA'} - \frac{1}{OA} = \frac{1}{f'}$$

    2)

$$\frac{d'}{d} = \frac{OA'}{OA},$$

    and
    3)

$$D = OA * Tan(\theta) + d,$$

Wobei

    * OA der Abstand zwischen dem Zentrum des fotosensitiven Elements des Infrarotsensors (4) und dem Zentrum der Objektivlinsen (20) ist und OA zwischen 0 und 100 mm liegt, vorzugsweise zwischen 5 und 50 mm und im besten Fall zwischen 10 und 30 mm.
    * OA' ist der Abstand zwischen dem Zentrum der Objektivlinse (29) und der Oberfläche der behandelten Haut (2), mit:

i) die Summe von OA' und OA sind grösser als oder gleich 10 mm vorzugsweise grösser oder gleich 30 mm und im besten Fall grösser oder gleich 50 mm, und
ii) die Summe von OA' und OA sind kleiner als oder gleich 500 mm vorzugsweise kleiner oder gleich 100 mm und im besten Fall kleiner oder gleich 75 mm.

* d ist der Durchmesser des fotosensitiven Elements des Infrarotsensors (4), wobei d zwischen 0,1 und 10 mm liegt, vorzugsweise zwischen 0,5 und 5 mm und im besten Fall zwischen 1 und 3 mm;
* θ steht für den Winkel des Sichtfeldes (5) des fotosensitiven Elements des infrarotsensors, wenn keine objektivlinse (20) vorhanden ist, wobei θ zwischen 10 und 80° liegt, vorzugsweise zwischen 15 und 60° und im besten Fall zwischen 20 und 40°;
* d' ist der Durchmesser des Abbildes des fotosensitiven Elements auf der Haut des Patienten. Dieses Abbild muss vollständig in der Oberfläche der mit dem dermatologischen Gerät behandelten Haut (2) liegen, wobei:

i) d+ Ist kleiner als 20 mm, vorzugsweise kleiner als 10 mm und im besten Fall kleiner als 5 mm, und
ii) d' ist grösser als 0,5 mm und vorzugsweise grösser als 1 mm;

* f+ Ist die Fokuslänge der Objektivlinse (20);
* D ist der Durchmesser der Objektivlinse (20), wobei D zwischen 3 und 100 mm liegen sollte, vorzugsweise zwischen 4 und 50 mm und im besten Fall zwischen 5 und 15 mm;

Wobei der Durchmesser und die für die Objektivlinse (20) bestimmte Position ermöglichen, dass das Sichtfeld (5) von zuvor erwähntem Infrarotsensor (4) derart fokussierbar ist, dass er vollständig auf dem Hautbereich (2), der vom Laserstrahl behandelt wird, liegt.

3. Das Gerät entsprechend Antrag 1 oder 2, wobei die Wellenlänge der Laserquelle von 1,1 bei 1,6 μm liegt, da 1,21 μm eine homogene Erwärmung in einer Tiefe von wenigstens 2 mm erlaubt.

4. Das Gerät entsprechend Antrag 1 bis 3, das sich darüber charakterisiert, dass zuvor erwähnte Möglichkeit der Kontrolle (13) über einen EIN/AUS-Schalter für die Laserquelle (1) funktioniert, wenn die vom infrarotsensor (4) im Bereich (2) der behandelten Haut gemessene Temperatur einen vorbestimmten Wert übersteigt.

5. Das Gerät entsprechend Antrag 1 bis 4, das sich darüber charakterisiert, dass die zuvor erwähnte Kontrollart (13) eine regulierende ist, die die Leistung der Laserquelle (1) anpassen kann, so dass die vom Infrarotsensor gemessene Temperatur in dem Bereich (2) der behandelten Haut zwischen 2 vorbestimmten Werten liegt.

6. Das Gerät entsprechend Antrag 1 bis 5 charakterisiert sich dadurch, dass die zuvor erwähnte Art der Filtration aus Silikon oder Germanium hergestellt ist.

7. Ein System einer dermatologischen Behandlung unter Verwendung eines Laserstrahls, dass sich darüber charakterisiert, dass es aus dem Gerät der oben erwähnten Anträge und den Arten der Interaktion (30, 32) zwischen der zuvor erwähnten Laserquelle und dem Bereich der behandelten Haut besteht. Die zuvor erwähnten Arten der Interaktion bestehen aus einem adhäsiven Medium (30), dass mit einer Art der Identifikation für die Kommunikation über Radiofrequenzen (31) konfiguriert Ist, wobei ein interface (32) mit den zuvor erwähnten Kontrollarten (13) verbunden Ist.

## Revendications

1. Un dispositif de traitement dermatologique par faisceau laser, comprenant :

- une source laser (1) apte à diriger un faisceau laser sur au moins une zone (2) de la surface de peau à traiter, ladite source laser permettant de délivrer, sur la surface de peau traitée, une fluence comprise entre 1 et 500 J/cm$^2$ de peau,
- des moyens de mesure sans contact sensibles à un rayonnement fonction de la température, configuré pour mesurer la température d'une surface de peau correspondant à la zone (2) de peau traitée, et
- des moyens d'asservissement (13) de ladite source laser auxdits moyens de mesure, dans lequel :
- lesdits moyens de mesure sans contact comprennent un capteur infrarouge (4) et une lentille de focalisation (20) apte à focaliser le champ de vision (5) dudit capteur infrarouge de sorte que la surface de peau contenue dans ledit champ de vision (5) soit intégralement comprise dans la zone (2) de peau traitée par le faisceau laser ;
- ladite tête comprend une cavité (24) munie d'une ouverture (26) qui est configurée pour être appliquée sur la surface de peau à traiter, le faisceau laser et le champ de vision des moyens de mesure sans contact traversant ladite cavité et débouchant dans ladite ouverture, ladite cavité étant partiellement fermée par un rebord intérieur (27) périphérique à ladite ouverture, ledit rebord intérieur (27) étant sensiblement plan et configuré pour être appliqué sur la surface de peau à traiter ;
- lequel dispositif comprend en outre des moyens de filtration disposés entre le capteur infrarouge et la surface de peau à traiter et constitués d'un matériau transparent dans la gamme des longueurs d'onde de 6 μm à 10 μm pour permettre la mesure de température, et opaque dans la gamme des longueurs d'onde de 0,6 μm à 2,5 μm.

2. Le dispositif selon la revendication 1, dans lequel la position et le diamètre de la lentille de focalisation (20) sont déterminés par la résolution des trois équations suivantes :

1)

$$\frac{1}{\overline{OA'}} - \frac{1}{\overline{OA}} = \frac{1}{f'}$$

2)

$$\frac{d'}{d} = \frac{\overline{OA'}}{\overline{OA}}$$

et
3)

$$D = OA * Tan(\theta) + d,$$

où

\* OA est la distance entre le centre de l'élément photosensible du capteur infrarouge (4) et le centre de la lentille de focalisation (20) avec OA comprise entre 0 et 100 mm, de préférence entre 5 et 50 mm, et de manière particulièrement préférée entre 10 et 30 mm ;
\* OA' est la distance entre le centre de la lentille de focalisation (20) et la surface de peau traitée (2) avec :

i) la somme de OA' et de OA est supérieure ou égale à 10 mm, de préférence supérieure ou égale à 30 mm, de manière particulièrement préférée supérieure ou égale à 50 mm, et
ii) la somme de OA' et de OA est inférieure ou égale à 500 mm, de préférence inférieure ou égale à 100 mm, et de manière particulièrement préférée inférieure ou égale à 75 mm :

\* d est le diamètre de l'élément photosensible du capteur infrarouge (4) avec d compris entre 0,1 et 10 mm, de préférence entre 0,5 et 5 mm, et de manière particulièrement préférée entre 1 et 3 mm ;
\* θ représente l'angle du champ de vision (5) de l'élément photosensible du capteur infrarouge (4) en l'absence de lentille de focalisation (20) avec θ compris entre 10 et 80 °, de préférence entre 15 et 60° et de manière particulièrement préférée entre 20 et 40° ;
\* d' est le diamètre de l'image de l'élément photosensible sur la peau du patient, laquelle image doit être intégralement comprise dans la surface de peau traitée (2) par le dispositif de traitement dermatologique avec :

i) d' est inférieur à 20 mm, de préférence d' est inférieur à 10 mm, et de manière particulièrement préférée inférieure à 5 mm, et
ii) d' est supérieur à 0,5 mm, de préférence d' est supérieur à 1 mm ;

\* f' est la focale de la lentille de focalisation (20) ;
\* D est le diamètre de la lentille de focalisation (20) avec D compris entre 3 et 100 mm, de préférence entre 4 et 50 mm, et de manière particulièrement préférée entre 5 et 15 mm;

dans lequel le diamètre et la position déterminés pour la lentille de focalisation (20) permettant d'obtenir une focalisation du champ de vision (5) dudit capteur infrarouge (4) de sorte qu'il soit entièrement compris dans la zone (2) de peau traitée.

3. Le dispositif selon l'une quelconque des revendications 1 ou 2, dans lequel la longueur d'onde de la source laser va de 1,1 à 1,6 μm, par exemple 1,21 μm, pour permettre un chauffage homogène jusqu'à une profondeur d'au moins 2 mm.

4. Le dispositif selon l'une quelconque des revendications 1 à 3, **caractérisé en ce que** lesdits moyens d'asservissement (13) sont des moyens par tout ou rien aptes à commander l'arrêt de la source laser (1) lorsque la température mesurée par le capteur infrarouge (4) sur la zone (2) de peau traitée dépasse une valeur prédéterminée.

5. Le dispositif selon l'une quelconque des revendications 1 à 4, **caractérisé en ce que** lesdits moyens d'asservissement (13) sont des moyens de régulation aptes à adapter la puissance de la source laser (1) de manière à maintenir la température mesurée par le capteur infrarouge (4) sur la zone (2) de peau traitée entre deux valeurs prédéterminées.

**6.** Le dispositif selon l'une quelconque des revendications 1 à 5, **caractérisé en ce que** lesdits moyens de filtration sont réalisés en silicium ou en germanium.

**7.** Un système de traitement dermatologique par faisceau lumineux, **caractérisé par le fait qu'**il comprend le dispositif selon l'une quelconque des revendications précédentes et des moyens d'interaction (30, 32) entre ladite source laser et la zone de peau à traiter, lesdits moyens d'interaction comprenant un support adhésif (30) muni de moyens d'identification configurés pour communiquer par radiofréquences (31) avec une interface (32) connectée avec lesdits moyens d'asservissement (13).

**Fig. 1**

**Fig. 2**

1

4

7

3

**Fig. 3**

3

T°1

2

T°2

5

**Fig. 4**

**Fig. 5**

**Fig. 6**

Fig. 7

Fig. 8

**Fig. 9**

**Fig. 10**

**Fig. 11(Temperature changes during laser treatment)**

**Fig. 12 ((Temperature changes during laser treatment))**

*Fig. 13 (Temperature changes during laser treatment)*

*Fig. 14 (Anti-burn mechanism set at 43°C)*

*Fig. 15 (Target temperature: 40°C)*

*Fig. 16*

*Fig. 17*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009071592 A
- WO 2008107563 A
- US 5409481 A
- US 20070179484 A
- US 20060041289 A1